# EUROPEAN PATENT APPLICATION

(11) **EP 2 602 614 A1**
(43) Date of publication of application: **12.06.2013**
(21) Application number: 10855614.3
(22) Date of filing: 04.08.2010
(51) Int. Cl.: G01N 27/00, G01N 21/35, G01N 27/06, G01N 27/22

(54) **APPARATUS FOR DETECTING FUEL CHARACTERISTICS**

(71) Applicant: Toyota Jidosha Kabushiki Kaisha, Toyota-shi, Aichi 471-8571 (JP)
(72) Inventor: AOKI, Keiichiro, Toyota-shi, Aichi 471-8571 (JP)
(74) Representative: Winter, Brandl, Fürniss, Hübner, Röss, Kaiser, Polte - Partnerschaft
(86) International application number: PCT/JP2010/063171
(87) International publication number: WO 2012/017524

(57) **Abstract**

Disclosed is a fuel property detection device that detects optical characteristics and electrical characteristics of fuel. The fuel property detection device detects the concentration of at least one component of fuel in accordance with the optical characteristics and detects the amount of metallic impurities contained in the fuel in accordance with the optical characteristics and with the electrical characteristics. The optical characteristics are represented, for instance, by a photorefractive index or a light transmittance that is obtained when light is incident on the fuel. The electrical characteristics are represented, for instance, by an electrical conductivity, a capacitance, or an AC impedance that is obtained when a voltage is applied between a pair of electrodes disposed to sandwich the fuel.

## Description

### Technical Field

The present invention relates to a fuel property detection device.

### Background Art

In recent years, a vehicle-mounted internal combustion engine capable of using alcohol, biofuel, synthetic fuel, or mixed fuel obtained by mixing one of these fuels with hydrocarbon fuel as an engine fuel has been researched and developed. The properties of the mixed fuel vary mainly with the type of a base fuel, that is, the hydrocarbon fuel, the type of the synthetic fuel or biofuel to be mixed, the mixing ratio of the hydrocarbon fuel and synthetic fuel or biofuel, and fuel quality changes caused by oxidative degradation. It is therefore preferred that factors affecting the fuel properties be accurately determined.

A fuel property detection device disclosed, for instance, in Patent Document 1 detects the fuel properties of a mixed fuel obtained by mixing biofuel with hydrocarbon fuel. The fuel property detection device has means for detecting the light transmittance, relative permittivity, and photorefractive index of the mixed fuel. The fuel property detection device calculates the concentration of RME (rape seed methyl ester), which is the biofuel in the mixed fuel, in accordance with the detected light transmittance of the mixed fuel, calculates the degree of oxidative degradation of the mixed fuel in accordance with the detected relative permittivity and with the calculated RME concentration, and calculates the type of light oil (light oil density) in accordance with the detected photorefractive index and with the calculated RME concentration and oxidative degradation.

### Prior Art Literature

### Patent Documents

- Patent Document 1:: JP-A-2009-281733
- Patent Document 2:: JP-A-2009-265079

### Summary of the Invention

### Problem to be Solved by the Invention

Metallic impurities, such as Fe and Ag, may be introduced into a mixed fuel, for instance, during its production process. In such an instance, it is necessary to correct, for example, a fuel injection amount and ignition timing in accordance with the amount of introduced metallic impurities. It is therefore preferred that the amount of metallic impurities be detected in addition to the concentration of each component in the mixed fuel and the degree of oxidative degradation of the mixed fuel. However, the invention disclosed in Patent Document 1 does not relate to the detection of the amount of metallic impurities.

The present invention has been made to solve the above problem. An object of the present invention is to provide a fuel property detection device that is improved to be capable of detecting the amount of metallic impurities introduced into a mixed fuel.

### Means for Solving the Problem

To achieve the foregoing object, first aspect of the present invention provides a fuel property detection device including:
optical characteristics detection means for detecting the optical characteristics of fuel;
electrical characteristics detection means for detecting the electrical characteristics of the fuel;
component concentration detection means for detecting the concentration of at least one component of the fuel in accordance with the optical characteristics; and
metallic impurities detection means for detecting the amount of metallic impurities contained in the fuel in accordance with the electrical characteristics and with the optical characteristics.

According to second aspect of the present invention, in the first invention, the optical characteristics detection means includes
first transmittance detection means for detecting a first transmittance, the first transmittance being the transmittance of light in a first wavelength region through the fuel, and
second transmittance detection means for detecting a second transmittance, the second transmittance being the transmittance of light in a second wavelength region through the fuel, the second wavelength region being different from the first wavelength region; and
wherein the component concentration detection means detects the concentration of the at least one component in accordance with the first transmittance and detects the concentration of water contained in the fuel in accordance with the second transmittance.

According to third aspect of the present invention, the metallic impurities detection means, in the second invention, detects the amount of metallic impurities in accordance with the first transmittance and/or the second transmittance and with the electrical characteristics.

According to fourth aspect of the present invention, in the first invention, the optical characteristics are represented by a photorefractive index or a light transmittance that is obtained when light is incident on the fuel.

According to fifth aspect of the present invention, in one of the first to fourth inventions, the electrical characteristics are represented by an electrical conductivity, a capacitance, or an AC impedance that is obtained when a voltage is applied between a pair of electrodes disposed to sandwich the fuel.

### Effects of the Invention

The first aspect of the present invention makes it possible not only to detect the concentration of a component in a fuel in accordance with optical characteristics, but also to detect the amount of metallic impurities in the fuel in accordance with electrical characteristics and optical characteristics. Therefore, when the amount of metallic impurities is to be detected, the concentration of a component that may affect the electrical characteristics can be taken into consideration to detect the amount of metallic impurities with increased accuracy.

A particular component and water in the fuel greatly vary their light transmittance from one wavelength region to another. Therefore, the second aspect of the present invention detects the concentration of a fuel component by detecting the light transmittance of the fuel with respect to light in the first wavelength region in which a particular component significantly affects the light transmittance, and detects the light transmittance of the fuel with respect to light in the second wavelength region in which the water significantly affects the light transmittance. This makes it possible to detect not only the concentration of the water in the fuel but also the concentration of the particular component in the fuel. As a result, fuel properties can be determined in greater detail.

According to the third aspect of the present invention, the amount of metallic impurities is detected in accordance with the electrical characteristics and with the first transmittance and/or the second transmittance, which are counted as the optical characteristics. The first transmittance is a value that varies with the concentration of a fuel component, whereas the second transmittance is a value that varies with the concentration of water in the fuel. Therefore, when the amount of metallic impurities is to be detected, the influence exerted by the concentration of a fuel component or water that may affect the electrical characteristics can be taken into consideration. Consequently, the amount of metallic impurities can be detected with increased accuracy.

### Brief Description of Drawings

[FIG.1] FIG. 1 is a diagram illustrating the configuration of a system including a fuel properties detection device and peripherals equipments according to a first embodiment of the present invention.
[FIG.2] FIG. 2 is a diagram illustrating the relationship between fuel density and photorefractive index of mixed fuel used in the first embodiment of the present invention.
[FIG.3] FIG. 3 is a diagram illustrating a relationship between electrical conductivity and metal ion concentration in the mixed fuel used in the first embodiment of the present invention.
[FIG.4] FIG. 4 is a flowchart illustrating a control routine that is executed by the system in the first embodiment of the present invention.
[FIG.5] FIG. 5 is a diagram illustrating a relationship between light transmittance and concentration of ethanol in other example of the first embodiment of the present invention.
[FIG.6] FIG. 6 is a diagram illustrating a relationship between wavelength of light and transmittance of light.
[FIG.7] FIG. 7 shows a relationship between the transmittance of the light, electrical conductivity, and amount of metallic impurities in a second embodiment of the present invention.
[FIG.8] FIG. 8 is a flowchart illustrating a control routine that is executed by the system in the second embodiment of the present invention.

### Mode for Carrying Out the Invention

Embodiments of the present invention will now be described with reference to the accompanying drawings. Elements that are shown in the drawings and identical or equivalent to each other are designated by the same reference numerals and will not be redundantly described or will be briefly described.

### First Embodiment

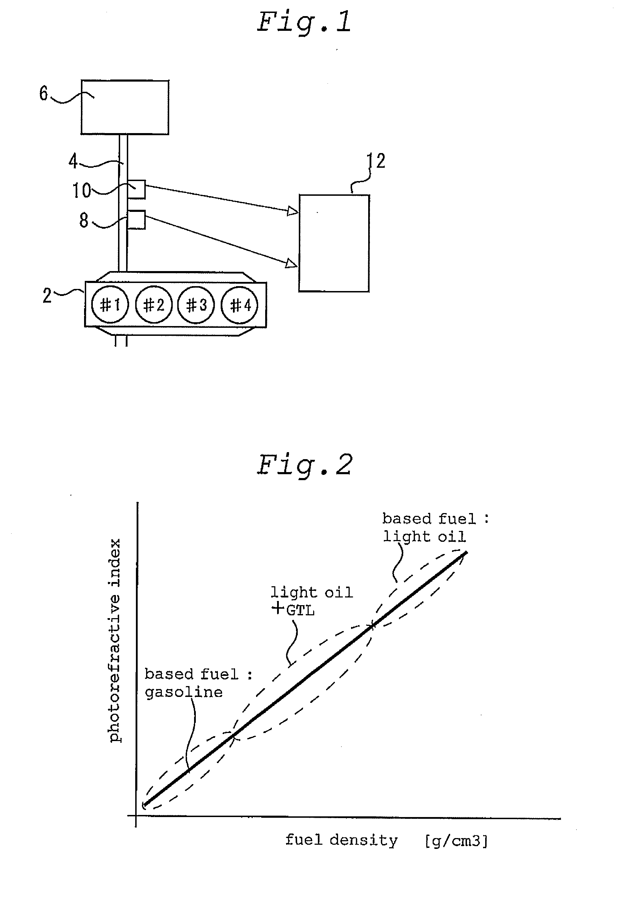

FIG. 1 is a diagram illustrating the configuration of a system according to a first embodiment of the present invention. As shown in FIG. 1, the system according to the first embodiment includes a vehicle-mounted internal combustion engine 2. Engine fuel used in the internal combustion engine 2 is gasoline, light oil, synthetic fuel such as GTL (gas to liquid) fuel, or a mixed fuel obtained by mixing some of these fuels.

A fuel injection valve (not shown) for injecting the fuel into each cylinder of the internal combustion engine 2 is connected to a fuel tank 6 through a fuel supply path 4. The mixed fuel is supplied to the fuel tank 6 for storage purposes.

An electrical characteristics detector 8 and an optical characteristics detector 10 are mounted in the fuel supply path 4. The electrical characteristics detector 8 is a sensor that generates an output signal in accordance with the electrical conductivity of the fuel distributed through the fuel supply path 4. Meanwhile, the optical characteristics detector 10 is a sensor that generates an output signal in accordance with the photorefractive index of the mixed fuel.

The electrical characteristics detector 8 has a pair of electrodes. The electrodes are disposed apart from each other so that they are exposed to the fuel in the fuel supply path 4. When a predetermined voltage is applied between the electrodes, the electrical characteristics detector 8 outputs the value of an electrical current that arises in accordance with the mixed fuel between the electrodes.

Meanwhile, the optical characteristics detector 10 includes a light-emitting element and a light-receiving element. The light-emitting element emits light having a predetermined wavelength. The light-receiving element receives reflected light. Further, the optical characteristics detector 10 has a space and reflective plates. The space is open, at least partly, so that the fuel in the fuel supply path 4 is distributed. The reflective plates are disposed so as to sandwich the space. The reflective plates reflect light emitted from the light-emitting element and transmitted through the fuel in the space toward the light-receiving element. In the optical characteristics detector 10, the light emitted from the light-emitting element is guided at a predetermined angle to fall on the mixed fuel in the space. The light incident on the mixed fuel and reflected from the reflective plates is received by the light-receiving element. The light-receiving element outputs a sensor signal indicative of the center of gravity of the received reflected light.

A control system for the internal combustion engine 2 according to the first embodiment includes an ECU (electronic control unit) 12. The ECU 12 is a control device that provides overall control of the entire system of the internal combustion engine 2. The output side of the ECU 12 is connected to various actuators, whereas the input side of the ECU 12 is connected to the electrical characteristics detector 8 and to the optical characteristics detector 10. The ECU 12 uses the outputs from these detectors as input signals to detect the electrical conductivity and photorefractive index of the mixed fuel. Further, the ECU 12 receives signals from various sensors to detect various information required for operating the internal combustion engine 2 and operates the various actuators in accordance with a predetermined control program. Although many actuators and sensors are connected to the ECU 12, they are not described in this document.

The light emitted from the light-emitting element of the optical characteristics detector 10 becomes incident at different positions of the light-receiving element because it is refracted at different angles in accordance with the fuel density of the mixed fuel. In other words, when a position at which the photorefractive index of the mixed fuel is minimized is regarded as a reference position, the distance from the reference position correlates with the photorefractive index. Therefore, when the light-receiving element detects a position at which the center of gravity of the reflected light is received, the ECU 12 can detect the photorefractive index of the mixed fuel.

FIG. 2 is a diagram illustrating the relationship between the fuel density and photorefractive index of the mixed fuel used in the first embodiment. As shown in FIG. 2, the photorefractive index increases with an increase in the fuel density of the mixed fuel and decreases with a decrease in the fuel density of the mixed fuel. It means that there is a certain correlation between the fuel density and photorefractive index of the mixed fuel. As such being the case, the relationship shown in FIG. 2 is measured in advance and stored in the ECU 12 in the form of a map or correlation function. The fuel density is calculated from the map or correlation function in accordance with the detected photorefractive index.

The fuel density detected as described above is used so that if the employed fuel is based, for instance, on gasoline, the mixing ratio of light oil to be mixed with the gasoline is detected in accordance with the density of the gasoline. If, on the other hand, the employed fuel is a mixed fuel obtained by mixing, for example, light oil with GTL or other synthetic fuel, the mixing ratio is detected in accordance with the density of the light oil. The concentration of each fuel component is detected in the above-described manner.

Meanwhile, when a predetermined voltage is applied between the electrodes of the electrical characteristics detector 8 in response to a control signal from the ECU 12, the electrical characteristics detector 8 outputs an electrical current value in accordance with the applied voltage. The ECU 12 can then detect the mixed fuel's electrical conductivity between the electrodes in accordance with the electrical current value.

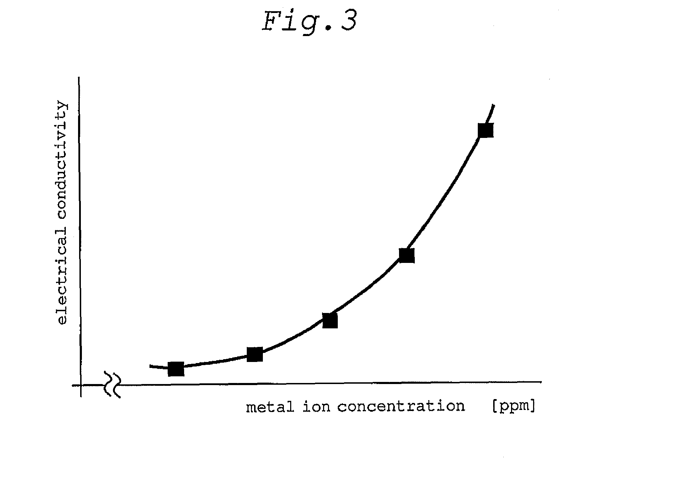

FIG. 3 is a diagram illustrating the relationship between the electrical conductivity and metal ion concentration in the mixed fuel used in the first embodiment. As shown in FIG. 3, there is such a correlation between the electrical conductivity and metal ion concentration that the electrical conductivity increases with an increase in the metal ion concentration in the mixed fuel and decreases with a decrease in the metal ion concentration in the mixed fuel. The metal ion concentration is a value that varies with the amount of metallic impurities.

The electrical conductivity also varies with the concentration of each fuel component of the mixed fuel. Therefore, if the photorefractive index is fixed, the electrical conductivity increases with an increase in the amount of metallic impurities. However, if the photorefractive index is not fixed, that is, if the concentration of a fuel component varies, the amount of metallic impurities varies even when the electrical conductivity remains unchanged.

There is a fixed correlation between the photorefractive index, the electrical conductivity, and the amount of metallic impurities. The system according to the first embodiment measures the relationship between the photorefractive index, the electrical conductivity, and the amount of metallic impurities, for instance, by conducting experiments in advance, and stores the measured relationship in the ECU 12 in the form of a three-dimensional map or correlation function. When fuel property detection is to be conducted during an actual control process, the amount of metallic impurities is detected from the map or the like in accordance with the photorefractive index and with the electrical conductivity.

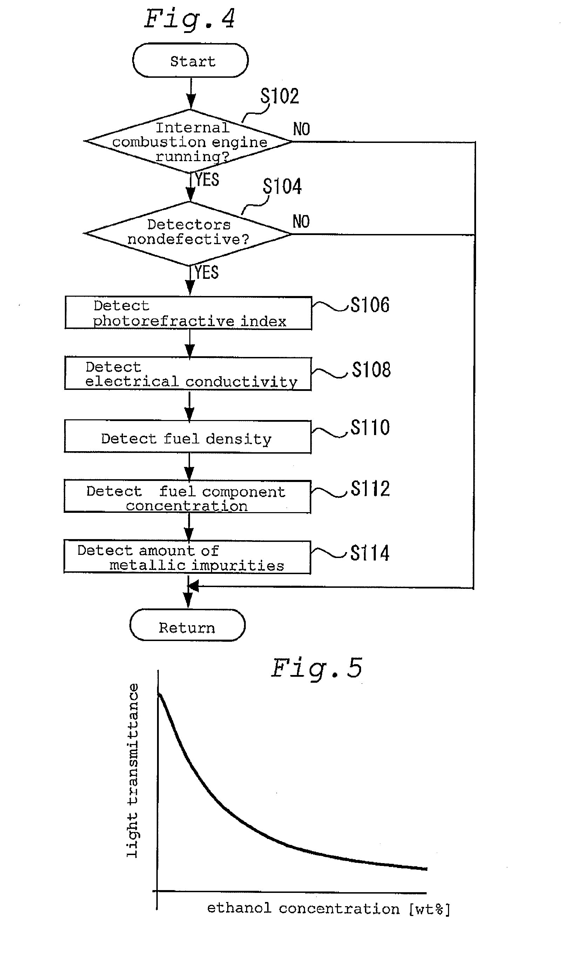

FIG. 4 is a flowchart illustrating a control routine that is executed by the ECU 12, which acts as a control device in the first embodiment. First of all, the routine shown in FIG. 4 judges whether the internal combustion engine 2 is started up and running (step S102). If the judgment result obtained in step S102 does not indicate that the internal combustion engine 2 is running, the routine terminates its process immediately.

If, on the other hand, the judgment result obtained in step S102 indicates that the internal combustion engine 2 is running, the routine proceeds to step S104 and judges whether the electrical characteristics detector 8 and the optical characteristics detector 10 are defective. If the judgment result obtained in step S104 indicates that the electrical characteristics detector 8 or the optical characteristics detector 10 is defective, the routine terminates its process.

If, on the other hand, the judgment result obtained in step S104 indicates that the electrical characteristics detector 8 and the optical characteristics detector 10 are both nondefective, the routine proceeds to step S106 and detects the photorefractive index. In step S106, the optical characteristics detector 10 turns on to let its light-emitting element emit light and let its light-receiving element detect the position of reflected light, generates an output signal indicative of the detected position, and inputs the generated output signal into the ECU 12. Upon receipt of the signal, the ECU 12 detects the photorefractive index.

Next, the routine proceeds to step S108 and detects the electrical conductivity. In step S108, the electrical characteristics detector 8 turns on so that the predetermined voltage is applied between the pair of electrodes. As a result, the value of an electrical current flowing in a circuit containing the pair of electrodes is output to the ECU 12. The ECU 12 detects the electrical conductivity in accordance with the received output.

Next, the routine proceeds to step S110 and computes the fuel density. More specifically, the fuel density is computed from the photorefractive index detected in step S104 in accordance with the relationship between the photorefractive index and fuel density that is stored in the ECU 12. Next, the routine proceeds to step S112 and calculates the concentration of a fuel component in accordance with the detected fuel density.

Next, the routine proceeds to step S114 and computes the amount of metallic impurities. The amount of metallic impurities is computed from the map or the like, which is stored in the ECU 12 to define the relationship between the photorefractive index, the electrical conductivity, and the amount of metallic impurities, in accordance with the photorefractive index detected in step S104 and with the electrical conductivity detected in step S106. Upon completion of step S114, the routine terminates its process.

As described above, the first embodiment makes it possible to determine the fuel density in accordance with a refractive index, which is one of the optical characteristics, detect the concentration of a fuel component, and detect the amount of metallic impurities in accordance with the photorefractive index and with the electrical conductivity, which is one of the electrical characteristics. Consequently, fuel properties essential to operate the internal combustion engine 2 can be accurately detected.

The first embodiment has been described on the assumption that when hydrocarbon fuel is used as the engine fuel, for example, the concentration of light oil in gasoline is detected or the ratio between light oil and synthetic fuel is detected as the concentration of a component. However, the present invention is not limited to such applications. The photorefractive index generally varies with the density of the hydrocarbon fuel. Therefore, if, for example, the employed mixed fuel is obtained by mixing gasoline with alcohol fuel or biofuel, and the density of the gasoline on which the mixed fuel is based and the type, for instance, of the alcohol fuel are known, the concentration of each fuel component of the mixed fuel can be detected in accordance with the fuel density calculated from the photorefractive index.

The first embodiment has also been described on the assumption that the optical characteristics detector 10 includes, for example, the light-emitting element, the light-receiving element, and the reflective plates and detects the photorefractive index by detecting the center of gravity of the reflected light. However, the configuration of the detector for detecting the photorefractive index is not limited to the aforementioned one. An alternative configuration for detecting the photorefractive index may be used.

Further, the first embodiment has been described on the assumption that the electrical characteristics detector 8 outputs the value of an electrical current when a voltage is applied to its pair of electrodes. However, the present invention is not limited to such a configuration. The electrical characteristics detector may have a different configuration as far as it outputs the electrical conductivity or other information about electrical characteristics.

Furthermore, the first embodiment has been described on the assumption that the optical characteristics detector 10 and the electrical characteristics detector 8 are both disposed in the fuel supply path 4. However, the present invention is not limited to such a configuration. Alternatively, they may be disposed, for instance, in the fuel tank 6. Another alternative is to dispose them in some other area through which a fuel whose properties need to be detected is distributed.

Moreover, the first embodiment has been described on the assumption that the photorefractive index is detected as one of the optical characteristics in order to detect the concentration of a fuel component. However, the present invention is not limited to the detection of the photorefractive index. An alternative is to detect some other optical characteristics and determine the concentration of a fuel component and the amount of metallic impurities in accordance with the detected optical characteristics. A case where light transmittance is detected as one of the optical characteristics to determine the concentration of a fuel component is described below.

FIG. 5 is a diagram illustrating the relationship between the light transmittance and the concentration of a fuel component. FIG. 5 shows a case where a mixed fuel obtained by mixing ethanol with gasoline is used. When light in a certain wavelength region is incident on the mixed fuel, the light transmittance varies with the concentration of alcohol in the mixed fuel as shown in FIG. 5. Further, the light transmittance correlates with the concentration of alcohol. Therefore, when a mixed fuel obtained by mixing alcohol with hydrocarbon fuel is used as the engine fuel, the concentration of alcohol can be detected by detecting the light transmittance.

Besides, the first embodiment has been described on the assumption that the amount of metallic impurities is detected in accordance with the photorefractive index and electrical conductivity. However, the present invention is not limited to such a method of detecting the amount of metallic impurities. The amount of metallic impurities affects the electrical characteristics (electrical conductivity, capacitance, AC impedance, etc.) of the mixed fuel, and there is a fixed correlation between the electrical characteristics of the mixed fuel and the amount of metallic impurities in the mixed fuel. Further, the electrical characteristics vary with the fuel concentration in the mixed fuel. Therefore, when the relationship between predefined optical characteristics (photorefractive index, light transmittance, etc.) of the mixed fuel, predefined electrical characteristics (electrical conductivity, capacitance, AC impedance, etc.) of the mixed fuel, and the amount of metallic impurities in the mixed fuel is measured in advance and stored in the ECU 12 in the form of a map or the like, the amount of metallic impurities in the mixed fuel can be determined by detecting its optical characteristics and electrical characteristics.

### Second Embodiment

The system according to a second embodiment of the present invention has the same configuration as the system shown in FIG. 1. The second embodiment differs from the first embodiment in that the concentration of water in the mixed fuel is taken into consideration when control is exercised to compute the amount of metallic impurities. The second embodiment will be described on the assumption that the employed mixed fuel is obtained by mixing gasoline with ethanol.

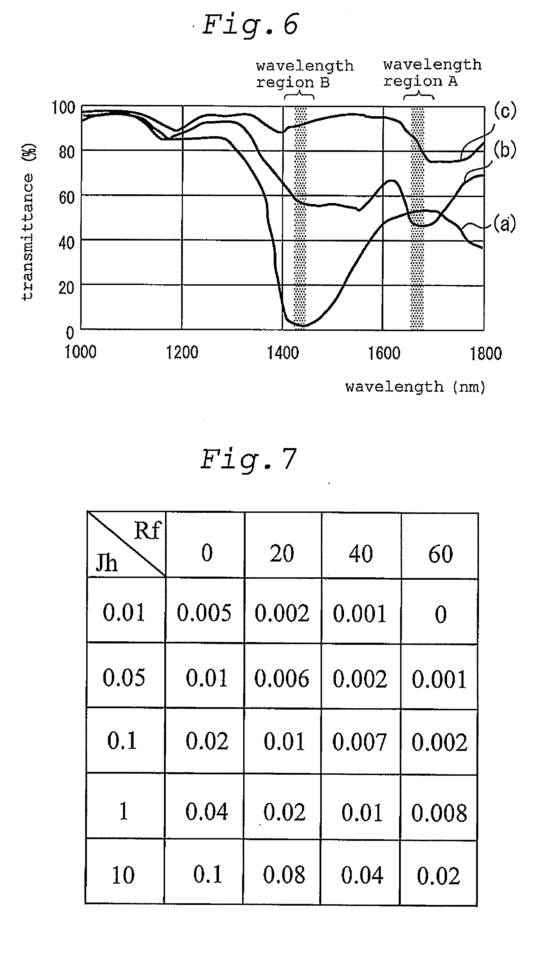

FIG. 6 is a diagram illustrating the relationship between the wavelength of light and the transmittance of the light. In FIG. 5, the horizontal axis represents the wavelength, whereas the vertical axis represents the transmittance. Further, in FIG. 6, curve (a) represents water, curve (b) represents ethanol, and curve (c) represents gasoline.

As is obvious from FIG. 6, the light transmittance of each component of the mixed fuel greatly varies with the wavelength of light incident on the mixed fuel. In wavelength region A (first wavelength region), for example, the light transmittance of ethanol greatly changes although the light transmittance of water (a) and of gasoline (c) slightly changes. In wavelength region B (second wavelength region), the light transmittance of water (a) significantly decreases although gasoline (c) and ethanol (b) do not significantly differ in light transmittance. Therefore, detecting the light transmittance when light in wavelength region A is incident on the mixed fuel and when light in wavelength region B is incident on the mixed fuel makes it possible to detect the concentration of ethanol and of water that greatly affects the changes in the light transmittance.

In a fuel property detection device according to the second embodiment, the relationship between the pre-measured transmittance (first transmittance) of the light in wavelength region A through the mixed fuel and the pre-measured concentration of ethanol as well as the relationship between the pre-measured transmittance (second transmittance) of the light in wavelength region B through the mixed fuel and the pre-measured concentration of water are respectively stored in the ECU 12 in the form of a map. When control is actually exercised to accomplish fuel property detection, the transmittance of the light in wavelength region A through the mixed fuel is detected, and then the concentration of ethanol in the mixed fuel is detected from the map in accordance with the detected light transmittance. Alternatively, the transmittance of the light in wavelength region B through the mixed fuel is detected, and then the concentration of water in the mixed fuel is detected from the map in accordance with the detected light transmittance.

Further, the fuel property detection device according to the second embodiment detects the amount of metallic impurities in accordance with the electrical conductivity and with the light transmittance (the transmittance of the light in wavelength region B) for detecting the concentration of water, which is determined as described above. FIG. 7 shows a map that relates to the second embodiment and defines the relationship between the transmittance of the light in wavelength region B, the electrical conductivity, and the amount of metallic impurities.

The electrical characteristics detected by the electrical characteristics detector 8 change particularly in accordance with variations of water included in the fuel and of fuel components. More specifically, when the light transmittance remains unchanged, the amount of metallic impurities increases with an increase in the electrical conductivity. When, on the other hand, the electrical conductivity remains unchanged, the amount of metallic impurities decreases with an increase in the light transmittance. In other words, there is a fixed correlation between the transmittance of the light in wavelength B, the electrical conductivity, and the amount of metallic impurities.

In the second embodiment, the fuel property detection device stores the aforementioned pre-measured correlation in the ECU 12 in the form of a three-dimensional map shown in FIG. 7 or in the form of a correlation function. The amount of metallic impurities is detected from the map or the like in accordance with the light transmittance and with the electrical conductivity.

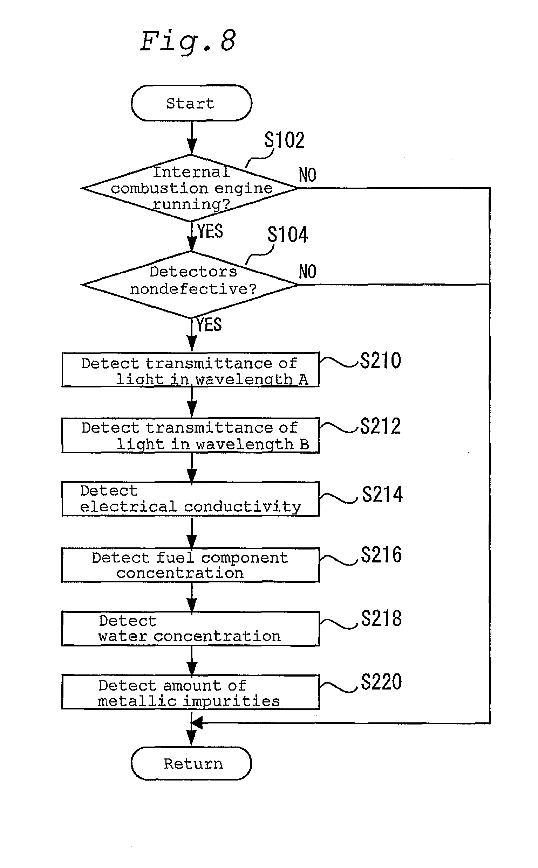

FIG. 8 is a flowchart illustrating a control routine that is executed by the system in the third embodiment. The routine shown in FIG. 8 is similar to the routine shown in FIG. 4 except that the former performs steps S210 and S212 after completion of step S104 and performs steps S216 to S220 after detecting the electrical conductivity in step S214.

More specifically, after each detector is found to be nondefective in step S104, the transmittance of the light in wavelength A is detected (step S210). As mentioned earlier, wavelength region A affects the light transmittance of ethanol. Next, the transmittance of the light in wavelength region B is detected (step S212). Wavelength region B particularly affects the light transmittance of water. Subsequently, the electrical conductivity is detected (step S214).

Next, the concentration of a fuel component is detected (step S216). More specifically, the concentration of ethanol in the mixed fuel is detected in accordance with the light transmittance detected in step S210. Next, the concentration of water is detected (step S218). Water concentration detection is accomplished by detecting the concentration of water in the mixed fuel in accordance with the transmittance of the light in wavelength region B, which was detected in step S212.

Next, the amount of metallic impurities is detected (step S220). The amount of metallic impurities is detected from the map (see FIG. 6) stored in the ECU 12 in accordance with the transmittance of the light in wavelength region B, which was detected in step S212, and with the electrical conductivity detected in step S214. Upon completion of step S220, the routine terminates its process.

As described above, the second embodiment detects the amount of metallic impurities from the transmittance of the light in wavelength region B, which affects the concentration of water, and from the electrical conductivity. Therefore, even when the mixed fuel used as the engine fuel contains alcohol or other fuel with which water readily mixes, the concentration of each component, including the concentration of water, can be detected with increased accuracy. In addition, the amount of metallic impurities can be detected on the basis of the influence of the concentration of each component. This makes it possible to detect the fuel properties of the mixed fuel with increased accuracy.

The second embodiment has been described on the assumption that the amount of metallic impurities is detected from the transmittance of the light in wavelength region B, which is used to detect the concentration of water, and from the electrical conductivity. However, the electrical characteristics may be affected not only by the concentration of water but also by the concentration of a fuel component such as alcohol. Therefore, when the amount of metallic impurities is to be detected, the concentration of each fuel component may be taken into consideration in addition to the concentration of water. More specifically, for example, the second embodiment may detect the amount of metallic impurities in accordance with the electrical conductivity and with the transmittance of the light in wavelength region A and in wavelength region B, or may consider only the concentration of each fuel component and detect the amount of metallic impurities in accordance with the electrical conductivity and with the transmittance of the light in wavelength region A. In such cases, too, the relationship between the transmittance of the light in wavelength region A and in wavelength region B, the electrical conductivity, and the amount of metallic impurities may be predefined in the form of a three-dimensional map or of a correlation function to detect metallic impurities in accordance with detected values.

Further, the second embodiment has been described on the assumption that the employed mixed fuel is obtained by mixing ethanol with gasoline. However, the present invention is not limited to the use of such a mixed fuel. The present invention is also applicable to a case where the employed mixed fuel is obtained by mixing another type of alcohol with biofuel or with hydrocarbon fuel or by mixing synthetic fuel with light oil or gasoline. In such cases, too, the concentration of a fuel component can be determined by detecting the light transmittance when light in a wavelength region that greatly differs in light transmittance from the other fuel components is incident on the mixed fuel. In addition, the amount of metallic impurities can be detected in accordance with the electrical conductivity and with the transmittance of the light in a wavelength region that is affected by a particular fuel component.

Moreover, the second embodiment has been described on the assumption that the amount of metallic impurities is detected in accordance with the light transmittance and with the electrical conductivity. However, the present invention is not limited to such a method of detecting the amount of metallic impurities. For example, when the concentration of biofuel or alcohol fuel is to be calculated in accordance with the photorefractive index as described in connection with the first embodiment, the relationship between the photorefractive index, the electrical conductivity, and the amount of metallic impurities can be measured in advance to calculate the amount of metallic impurities as well as the photorefractive index.

When the number, quantity, amount, range, or other numerical attribute of an element is mentioned in the above description of the foregoing embodiments, the present invention is not limited to the mentioned numerical attribute unless it is expressly stated or clearly defined in principle. Further, structures and steps described in connection with the embodiments are not necessarily essential to the present invention unless they are expressly stated or clearly defined in principle.

### Description of Notations

| | |
|---|---|
| 2 | internal combustion engine |
| 4 | fuel supply path |
| 6 | fuel tank |
| 8 | electrical characteristics detector |
| 10 | optical characteristics detector |

## Claims

1. A fuel property detection device comprising:
optical characteristics detection means for detecting the optical characteristics of fuel;
electrical characteristics detection means for detecting the electrical characteristics of the fuel;
component concentration detection means for detecting the concentration of at least one component of the fuel in accordance with the optical characteristics; and
metallic impurities detection means for detecting the amount of metallic impurities contained in the fuel in accordance with the electrical characteristics and with the optical characteristics.

2. The fuel property detection device according to claim 1, wherein the optical characteristics detection means includes
first transmittance detection means for detecting a first transmittance, the first transmittance being the transmittance of light in a first wavelength region through the fuel, and
second transmittance detection means for detecting a second transmittance, the second transmittance being the transmittance of light in a second wavelength region through the fuel, the second wavelength region being different from the first wavelength region; and
wherein the component concentration detection means detects the concentration of the at least one component in accordance with the first transmittance and detects the concentration of water contained in the fuel in accordance with the second transmittance.

3. The fuel property detection device according to claim 2, wherein the metallic impurities detection means detects the amount of metallic impurities in accordance with the first transmittance and/or the second transmittance and with the electrical characteristics.

4. The fuel property detection device according to claim 1, wherein the optical characteristics are represented by a photorefractive index or a light transmittance that is obtained when light is incident on the fuel.

5. The fuel property detection device according to any one of claims 1 to 4, wherein the electrical characteristics are represented by an electrical conductivity, a capacitance, or an AC impedance that is obtained when a voltage is applied between a pair of electrodes disposed to sandwich the fuel.
